# FASCICULE DE BREVET EUROPEEN

(11) **EP 3 587 384 B1**
(45) Date de publication et mention de la délivrance du brevet: **18.09.2024**
(21) Numéro de dépôt: 19181580.2
(22) Date de dépôt: 21.06.2019
(51) Int. Cl.: C07C 7/00, C07C 15/08, C07C 7/13, B01D 15/18, C07C 7/04, B01D 3/14

(54) **PROCÉDÉ DE PRODUCTION DE PARAXYLÈNE UTILISANT UNE ÉTAPE EN LIT MOBILE SIMULÉ, ET UNE ÉTAPE DE FRACTIONNEMENT VIA UNE COLONNE DE 3 COUPES**
HERSTELLUNGSVERFAHREN VON PARAXYLEN MIT EINER PHASE IM SIMULIERTEN FLIESSBETT UND EINER FRAKTIONIERUNGSPHASE ÜBER EINE SÄULE MIT 3 SCHNITTEN
METHOD FOR PRODUCING PARAXYLENE USING A STEP IN A SIMULATED MOVING BED, AND A FRACTIONATION STEP VIA A 3-SECTION COLUMN

(30) Priorité: 29.06.2018 FR 1856050
(43) Date de publication de la demande: 01.01.2020
(73) Titulaire: AXENS, 92508 Rueil-Malmaison Cedex (FR)
(72) Inventeur: PREVOST, Isabelle, 92508 Rueil-Malmaison Cedex (FR); PIGOURIER, Jérôme M., 92508 Rueil-Malmaison Cedex (FR); MARTIN, Pierre-Yves, 92508 Rueil-Malmaison Cedex (FR); COTTE, Arnaud, 92508 Rueil-Malmaison Cedex (FR)
(74) Mandataire: IFP Energies nouvelles

(56) Documents cités:
- WO-A1-2013/089902
- WO-A1-2016/175898
- FR-A1- 2 862 638
- US-A1- 2014 179 975

## Description

### DOMAINE TECHNIQUE

Le paraxylène est principalement utilisé pour les productions d'acide téréphtalique et de résines polyéthylène téréphtalate, pour fournir des textiles synthétiques, des bouteilles, et plus généralement des matières plastiques.

La présente invention concerne un procédé de production de paraxylène à haute pureté mettant en oeuvre un enchaînement spécifique d'étapes permettant d'atteindre de forte production de paraxylène.

### ART ANTERIEUR

La production de paraxylène haute pureté mettant en oeuvre une étape de séparation par adsorption est bien connue de l'art antérieur. De manière industrielle, ladite étape est réalisée au sein d'un enchaînement de procédés dit « boucle C8-aromatique » ou encore « boucle de xylène ». Cette « boucle C8-aromatique » inclut une étape d'élimination des composés lourds (c'est-à-dire contenant plus de 9 atomes de carbone, notés C9+) dans une colonne de distillation appelée « colonne des xylènes ».

Le flux de tête de cette colonne, qui contient les isomères en C8-aromatiques, est ensuite envoyé dans le procédé de séparation du paraxylène qui est généralement une étape de séparation par adsorption en lit mobile simulé.

L'extrait obtenu à l'issue de l'étape de séparation par adsorption en lit mobile simulé, qui contient le paraxylène est ensuite distillé au moyen d'une colonne d'extrait puis d'une colonne toluène, pour obtenir du paraxylène de haute pureté.

Le raffinat obtenu à l'issu de l'étape de séparation par adsorption en lit mobile simulé, riche en métaxylène, orthoxylène et éthylbenzène, après une étape d'élimination du désorbant par distillation, est mis en oeuvre dans une étape d'isomérisation, permettant l'obtention d'un mélange dans lequel la proportion des xylènes (ortho-, méta-, para- xylènes) est pratiquement à l'équilibre thermodynamique, et appauvri en éthylbenzène. Ce mélange est à nouveau envoyé dans la « colonne des xylènes » avec la charge fraiche pour la production de paraxylène.

L'art antérieur propose de nombreuses variantes de ce schéma mettant en oeuvre une ou plusieurs étape de séparation (par adsorption, cristallisation, distillation ou par membrane) et/ou une ou plusieurs étape d'isomérisation en phase gazeuse (convertissant l'éthylbenzène par isomérisation en xylènes ou par désalkylation en benzène), ou en phase liquide (ne convertissant pas l'éthylbenzène). L'une des variations proposées dans l'art antérieur consiste à réaliser l'isomérisation en deux étapes distinctes. La première étape permet d'isomériser l'éthylbenzène contenu dans le raffinat riche en métaxylène, orthoxylène et éthylbenzène. L'effluent de cette première section d'isomérisation, appauvri en éthylbenzène, est envoyé après fractionnement dans une deuxième section comprenant la séparation du paraxylène par adsorption suivie d'une deuxième étape d'isomérisation des xylènes en paraxylène.

Le document FR2862638 décrit un procédé de production de PX, en particulier, un procédé utilisant la méthylation du toluène et / ou du benzène pour produire sélectivement du PX, dans lequel les flux ayant différentes quantités d'EB sont traités séparément dans la récupération du PX, ou le lit mobile simulé permet l'obtention de trois flux.

L'optimisation des catalyseurs d'isomérisation et des conditions opératoires permet de limiter les réactions secondaires indésirables. Ainsi, il est établi que pour favoriser la conversion de l'éthylbenzène en xylènes, il est préférable de réaliser l'isomérisation en phase vapeur à haute température. De même, il est connu que l'isomérisation en phase liquide à basse température minimise les réactions parasites de craquage, de transalkylation et de dismutation, et limite également la conversion de l'éthylbenzène.

Les procédés de production de paraxylène à haute pureté mettant en oeuvre deux étapes d'isomérisation sont plus complexes et conduisent à des gains de production de paraxylène limités. En effet pour atteindre des taux de conversion de l'éthylbenzène suffisamment élevés, les conditions opératoires de la première étape d'isomérisation en phase gazeuse sont sévères et s'accompagne de réactions secondaires qui conduisent à des pertes en C8 aromatiques ce qui impacte fortement le rendement global en paraxylène.

Le brevet WO2016175898A1 décrit un procédé de production de paraxylène à partir d'une charge d'hydrocarbures, utilisant deux étapes de séparation en lit mobile simulé, et deux étapes d'isomérisation. L'inconvénient de ce procédé est de nécessiter deux étapes de séparation en lit mobile simulé ce qui entraine une augmentation importante du coût de production.

Les brevets FR 3023840 et FR3023841 décrivent des procédés alternatifs comprenant deux étapes de séparation en lit mobile simulé et étapes unités d'isomérisation dans lesquels les deux étapes d'isomérisation sont opérées en séries ou en parallèles sur le circuit du raffinat de la première unité de séparation en lit mobile simulé.

Le brevet FR 3023842 décrit un procédé alternatif comprenant une étape d'adsorption en lit mobile simulé et deux étapes d'isomérisation, en phase gaz d'une part et en phase liquide d'autre part, dans lequel les deux unités d'isomérisation sont alimentées par la même charge issue du fractionnement de l'effluent de l'unité d'adsorption.

Dans le domaine de l'invention, l'homme du métier cherche constamment à limiter les coûts d'investissement et les coûts opératoires de fonctionnement de la boucle xylène mise en oeuvre tout en augmentant la quantité de paraxylène à haute pureté obtenu.

De manière surprenante, la demanderesse a découvert que la combinaison dans un procédé de production de paraxylène à haute pureté d'une étape de séparation par adsorption en SMB, à une étape ultérieure de séparation par distillation dans une première colonne de distillation 3 coupes produisant au moins deux raffinats et de deux étapes isomérisations, permet d'améliorer le rendement global en paraxylène de la boucle aromatique et de minimiser l'impact économique.

Un avantage du procédé selon la présente invention est d'atteindre une forte production de paraxylène, moyennant de faibles contreparties d'augmentation de capacité, contrairement aux procédés de l'art antérieur.

Un autre avantage de l'invention est de présenter une grande flexibilité pouvant être mise à profit en opération, permettant ainsi de pouvoir s'adapter à l'évolution des performances catalytiques pour chaque étape d'isomérisation mise en oeuvre.

### DEFINITIONS & ABREVIATIONS

Dans l'ensemble de la description les termes ou abréviations ci-après ont le sens suivant.

Il est précisé que, dans toute cette description, l'expression « compris(e) entre ... et ... » doit s'entendre comme incluant les bornes citées.

On désigne par l'abréviation EB, l'éthylbenzène.

On désigne par l'abréviation PX, le paraxylène.

On désigne par l'abréviation OX, l'orthoxylène.

On désigne par l'abréviation MX, le métaxylène.

On entend par xylènes (encore noté XYL), un mélange d'au moins deux isomères de xylènes choisis parmi l'orthoxylène, le métaxylène, et le paraxylène.

On désigne par l'abréviation anglaise SMB, un lit mobile simulé.

On entend par hydrocarbures en C9+, des hydrocarbures contenant au moins 9 atomes de carbone.

On entend par hydrocarbures en C8+, des hydrocarbures contenant au moins 8 atomes de carbone.

On désigne par C8Aromatiques, encore noté C8A, les hydrocarbures aromatiques constitués de 8 atomes de carbone soit EB, PX, OX, MX et de préférence EEB, OX, MX.

On entend par raffinat un mélange de C8A appauvri en PX et pouvant contenir du désorbant, c'est-à-dire présentant une teneur massique en PX inférieur à 2,0%, de préférence inférieur à 1,5% et de manière préférée à 1,0%..

On entend par exempt au sens de la présente invention, une teneur massique en un composé donné par rapport à la masse total de la fraction considérée, par exemple en EB, inférieure à 0,5% poids, de préférence inférieur à 0,1% et de manière préférée inférieure à 0,01%.

On entend par quantité résiduelle d'un composé donné, une quantité dont la teneur massique par rapport à la masse total de la fraction considérée est inférieure à 5,0% poids, de préférence comprise entre 5,0 et 1,0, de préférence comprise entre 4,0 et 1,0, et de manière préférée entre 3,0 et 1,0% poids.

Dans la présente invention, les termes effluents, raffinats, flux et fractions sont employés de façon équivalentes.

On entend par colonne de distillation 2 coupes et 3 coupes, une colonne de distillation permettant l'obtention de 2 et 3 fractions respectivement.

### DESCRIPTION SOMMAIRE DE L'INVENTION

La présente invention concerne donc un procédé de production de paraxylène à partir d'une charge contenant des xylènes, de l'éthylbenzène et des hydrocarbures en C9+, comprenant
- une unique étape A de séparation en lit mobile simulé de ladite charge mise en oeuvre avec une zéolithe comme adsorbant et un désorbant, à une température comprise entre 20 et 250°C, sous une pression comprise entre 1,0 et 2,2 MPa, et avec un rapport volumique du désorbant sur la charge dans l'unité de séparation en lit mobile simulé est compris entre 0,4 et 2,5 ; ladite étape A permettant l'obtention de
   ▪ une première fraction A1 contenant un mélange de paraxylène et de désorbant, et
   ▪ d'au moins une deuxième fraction A2 ou A21 contenant de l'éthylbenzène (EB), de l'orthoxylène (OX) et du métaxylène (MX) et du désorbant,
- une étape B de fractionnement par distillation dans au moins une première colonne de distillation B_C3 de ladite deuxième fraction issue de l'étape A permettant l'obtention de 3 fractions :
   ▪ une première fraction B2 contenant de l'EB, de l'OX et du MX,
   ▪ une seconde fraction B3 contenant de l'OX et du MX, et
   ▪ une troisième fraction B42 contenant du désorbant,
dans lequel l'étape A de séparation permet l'obtention d'une troisième fraction A22 appauvri en EB contenant un mélange du MX, de l'OX et du désorbant, lesdites fractions A21 et A22 étant envoyées dans ladite étape B de fractionnement, la troisième fraction (A22) issue de l'étape A est engagée dans une seconde colonne de distillation (B-C4) permettant l'obtention d'une fraction (B31) exempte de désorbant contenant du MX et de l'OX, et d'une fraction B43 constituée de désorbant,la seconde fraction de (B3) est engagée à l'étape B de fractionnement dans une seconde colonne de distillation (B-C4), permettant l'obtention d'une fraction (B31) exempte de désorbant contenant du MX, et de l'OX, et une fraction B43 constituée de désorbant,la troisième fraction (A22) issue de l'étape A est introduite dans la seconde colonne de distillation (B-C4) en dessous du point d'injection latéral de la seconde fraction (B3) issue de la première colonne de distillation (B-C3).

De préférence, la première fraction B2 issue de l'étape B présente une teneur en EB supérieure à la teneur en EB de la seconde fraction B3 issue de la première colonne B_C3 mise en oeuvre à l'étape B. De préférence, la teneur en EB de la première fraction est supérieur d'au moins 1,0% par rapport à la seconde fraction.

De préférence, la colonne de distillation mise en oeuvre à l'étape B comprend entre 30 et 80 plateaux théoriques.

L 'étape A de séparation permet l'obtention en outre d'une troisième fraction A22 appauvri en EB contenant un mélange du MX, de l'OX et du désorbant, lesdites fractions A21 et A22 étant envoyées dans ladite étape B de fractionnement.

La troisième fraction A22 issue de l'étape A est engagée dans une seconde colonne de distillation B-C4 permettant l'obtention d'une fraction B31 exempte de désorbant contenant du MX et de l'OX, et d'une fraction B43 constituée de désorbant.

La seconde fraction de B3 est engagée à l'étape B de fractionnement dans une seconde colonne de distillation B-C4, permettant l'obtention d'une fraction B31 exempte de désorbant contenant du MX, et de l'OX, et une fraction B43 constituée de désorbant.

La troisième fraction A22 issue de l'étape A est introduite dans la seconde colonne de distillation B-C4 en dessous du point d'injection latéral de la seconde fraction B3 issue de la première colonne de distillation B-C3.

Avantageusement, l'étape B de distillation met en oeuvre une colonne comprenant une paroi interne.

Avantageusement, le procédé comprend en outre une étape C d'isomérisation en phase vapeur de la première fraction B2 issue d'une étape de fractionnement contenant de l'EB, de l'OX et du MX.

Selon une variante non incluse dans l'invention, le procédé comprend en outre une étape D d'isomérisation en phase liquide de la deuxième fraction B3 contenant de l'OX et du MX issue de la première colonne de distillation B_C3 mise en oeuvre à étape B de fractionnement.

Avantageusement, le procédé comprend en outre une étape D d'isomérisation en phase liquide de la fraction B31 contenant de l'OX et du MX issue de la seconde colonne de distillation B_C4 mise en oeuvre à l'étape de fractionnement.

Avantageusement, l'étape C d'isomérisation en phase vapeur est mise en oeuvre à une température supérieure à 300°C, une pression inférieure à 4,0 MPa, une vitesse spatiale inférieure à 10,0 h⁻¹, rapport molaire hydrogène sur hydrocarbure inférieur à 10,0, et en présence d'un catalyseur comportant au moins une zéolithe présentant des canaux dont l'ouverture est définie par un anneau à 10 ou 12 atomes d'oxygène (10 MR ou 12 MR), et au moins un métal du groupe VIII de teneur comprise entre 0,1 et 0,3% poids.

Avantageusement, le catalyseur mise en oeuvre à l'étape C comprend de 1 à 70% poids d'une zéolithe de type structural EUO, ladite zéolithe comprenant du silicium et au moins un élément T choisi de préférence parmi l'aluminium et le bore dont le rapport Si/T est compris entre 5 et 100.

Avantageusement, l'étape D d'isomérisation en phase liquide est mise en oeuvre à une température inférieure à 300°C, une pression inférieure à 4,0 MPa, une vitesse spatiale inférieure à 5,0 h⁻¹ et de préférence comprise entre 2,0 et 4,0 h⁻¹, et en présence d'un catalyseur comportant au moins une zéolithe présentant des canaux dont l'ouverture est définie par un anneau à 10 ou 12 atomes d'oxygène (10 MR ou 12 MR), de préférence ladite zéolithe est de type ZSM-5.

### PRESENTATION SUCCINCTE DES FIGURES

La figure 1 représente un schéma général d'une boucle Xylènes mettant en oeuvre une étape de séparation par adsorption, une étape de fractionnement, une étape C d'isomérisation en phase vapeur et une étape D d'isomérisation en phase liquide.
La figure 2a représente une étape B de distillation selon l'art antérieur du raffinat issu de l'étape A.
La figure 2b représente une première variante de l'étape B de distillation du raffinat issu de l'étape A.
La figure 2c représente une seconde variante de l'étape B de distillation du raffinat issu de l'étape A.
La figure 3a représente l'étape B de distillation de deux raffinats issus de l'étape A selon l'art antérieur.
La figure 3b représente une variante selon l'invention, de l'étape B de distillation de deux raffinats issus de l'étape A.

### DESCRIPTION DETAILLEE DE L'INVENTION

Les caractéristiques et avantages du procédé selon l'invention, apparaîtront à la lecture de la description ci-après, d'exemples non limitatifs de réalisations, en se référant aux numérotations des modes de réalisation illustrés dans les figures selon l'invention.

Dans le sens de la présente invention, les différents modes de réalisation présentés peuvent être utilisés seul ou en combinaison les uns avec les autres, sans limitation de combinaison.

La présente invention concerne donc un procédé de production de paraxylène à partir d'une charge contenant des xylènes, de l'éthylbenzène et des hydrocarbures en C9+, comprenant
- une unique étape A de séparation en lit mobile simulé de ladite charge mise en oeuvre avec une zéolithe comme adsorbant et un désorbant, à une température comprise entre 20 et 250°C, sous une pression comprise entre 1,0 et 2,2 MPa, et avec un rapport volumique du désorbant sur la charge dans l'unité de séparation en lit mobile simulé est compris entre 0,4 et 2,5 ; ladite étape A permettant l'obtention de
   ▪ une première fraction A1 contenant un mélange de paraxylène et de désorbant, et
   ▪ d'au moins une deuxième fraction A2 ou A21 contenant de l'éthylbenzène (EB), de l'orthoxylène (OX) et du métaxylène (MX) et du désorbant,
- une étape B de fractionnement par distillation dans au moins une première colonne de distillation B_C3 de ladite deuxième fraction issue de l'étape A permettant l'obtention de 3 fractions :
   ▪ une première fraction B2 contenant de l'EB, de l'OX et du MX,
   ▪ une seconde fraction B3 contenant de l'OX et du MX, et
   ▪ une troisième fraction B42 contenant du désorbant, dans lequel l'étape A de séparation permet l'obtention d'une troisième fraction A22 appauvri en EB contenant un mélange du MX, de l'OX et du désorbant, lesdites fractions A21 et A22 étant envoyées dans ladite étape B de fractionnement,la troisième fraction (A22) issue de l'étape A est engagée dans une seconde colonne de distillation (B-C4) permettant l'obtention d'une fraction (B31) exempte de désorbant contenant du MX et de l'OX, et d'une fraction B43 constituée de désorbant,la seconde fraction de (B3) est engagée à l'étape B de fractionnement dans une seconde colonne de distillation (B-C4), permettant l'obtention d'une fraction (B31) exempte de désorbant contenant du MX, et de l'OX, et une fraction B43 constituée de désorbant,la troisième fraction (A22) issue de l'étape A est introduite dans la seconde colonne de distillation (B-C4) en dessous du point d'injection latéral de la seconde fraction (B3) issue de la première colonne de distillation (B-C3).

Le procédé selon l'invention permet ainsi d'obtenir à l'issu de l'étape de fractionnement deux fractions ayant des proportions différentes en C8A et ainsi d'augmenter la proportion de l'isomérisation des xylènes en PX réalisée par le catalyseur opérant en phase liquide et permet de limiter les pertes en C8A.

### Etape A de séparation en lit mobile simulé

Selon l'invention, le procédé comprend une unique étape A de séparation en lit mobile simulé (SMB) d'une charge contenant des xylènes, de l'éthylbenzène et des hydrocarbures en C9+. Ladite étape de séparation en SMB est mise en œuvre avec une zéolithe comme adsorbant et un désorbant et permettant l'obtention d'au moins deux fractions, une fraction A1 encore appelée « extrait » contenant un mélange de paraxylène(PX) et de désorbant, une fraction A2 encore appelée « raffinat » contenant de l'éthylbenzène (EB), de l'orthoxylène (OX), du métaxylène (MX) et du désorbant.

L'étape de séparation est mise en oeuvre dans une unité opérant en lit mobile simulé dans au moins une colonne de séparation contenant une pluralité de lits interconnectés et faisant circuler du désorbant en boucle fermée dont sont issus deux fractions :
- la première est un extrait A1 comprenant, de préférence constituée, de paraxylène et du désorbant de telles sorte qu'après fractionnement pour éliminer le désorbant le PX atteigne une pureté commerciale de minimum 99,0 % et préférentiellement 99,9 % poids. Avantageusement, l'extrait A1 présente au moins 30% poids de la masse total de l'extrait.
- la deuxième fraction est un raffinat A2 qui contient de l'éthylbenzène (EB), du métaxylène et de l'orthoxylène et du désorbant. De préférence, le raffinat est appauvri en paraxylène c'est-à-dire que la teneur massique en PX dans ledit raffinat est inférieure à 1,0 %, et de préférence inférieure à 0,5 %.

Selon l'invention, trois fractions sont obtenues à l'unique étape de séparation en SMB,
- la première est un extrait A1 comprenant, de préférence constitué, de paraxylène et du désorbant, après fractionnement de ce raffinat le PX est obtenu aux spécifications commerciales. Avantageusement, l'extrait A1 présente au moins 30% poids de la masse total de l'extrait,
- deux fractions A21 et A22 appauvries de paraxylène qui contiennent dans des proportions variables un mélange de EB, MX, OX, et du désorbant.

Dans ce mode de réalisation, les fractions A21 et A22 présentent des proportions en EB, MX, OX différentes de telle sorte que la teneur en EB de la coupe CA8 de la fraction A21 est supérieure à la teneur en EB de la coupe CAS de la fraction A22. De préférence, la fraction A21 appauvrie en PX contient un mélange de EB, de MX, OX, et de désorbant, de préférence l'EB est en quantité résiduelle. De préférence, la fraction A22 appauvrie en EB et de PX contient un mélange MX,OX, et du désorbant.

De préférence, l'adsorbant utilisé dans l'unité de séparation en lit mobile simulé est une zéolithe X échangée au baryum ou une zéolithe Y échangée au potassium ou une zéolithe Y échangée au baryum et au potassium.

Dans un mode de réalisation, le désorbant contenu dans la charge traitée par le procédé selon l'invention est dit lourd c'est-à-dire avec un point d'ébullition supérieur à celui des xylènes.

Dans un autre mode de réalisation, le désorbant contenu dans la charge traitée par le procédé selon l'invention est dit léger c'est-à-dire avec un point d'ébullition inférieur à celui des xylènes.

De préférence, le désorbant utilisé dans l'unité de séparation en lit mobile simulé est choisi parmi le paradiéthylbenzène, le toluène, le paradifluorobenzène ou des diéthylbenzènes seul ou en mélange.

De préférence, le rapport volumique du désorbant sur la charge dans l'unité de séparation en lit mobile simulé est compris entre 0,4 et 2,5, et de préférence compris entre 0,5 et 1,5.

De préférence, l'étape A de séparation en lit mobile simulé est mise en oeuvre à une température comprise entre 20°c et 250°C, de préférence entre 90°C et 210°C, et de manière encore préférée entre 160°C et 200°C, et sous une pression comprise entre 1,0 et 2,2 MPa, et de préférence entre 1,2 et 2,0MPa.

De préférence, l'adsorbeur contient une pluralité de lits, interconnectés et répartis sur plusieurs zones délimitées par les injections de la charge et du désorbant, ainsi que des soutirages de l'extrait, et du ou des raffinats. Selon le nombre de raffinat l'adsorbeur contiendra préférentiellement 18 à 15 lits.

Selon un mode particulier de réalisation, le nombre total de lits de l'unité de séparation (SMB) est compris entre 10 et 30 lits, et de manière préférée, entre 15 et 18 lits répartis sur un ou plusieurs adsorbeurs, le nombre de lits étant ajusté de manière à ce que chaque lit ait une hauteur comprise entre 0,70 m et 1,40 m.

Selon un mode particulier de réalisation (ALT 0, ALT 1), la répartition de la quantité de solide adsorbant dans chaque zone de l'unité de séparation (SMB) est la suivante :
- la quantité de solide adsorbant en zone 1 est de 17%±5%,
- la quantité de solide adsorbant en zone 2 est de 42%±5%,
- la quantité de solide adsorbant en zone 3 est de 25%±5%,
- la quantité de solide adsorbant en zone 4 est de 17%±5%.

Selon un autre mode particulier de réalisation (ALT 2), la répartition de la quantité de solide adsorbant dans chaque zone de l'unité de séparation (SMB) est la suivante :
- la quantité de solide adsorbant en zone 1 est de 18%±8%,
- la quantité de solide adsorbant en zone 2 est de 41%±8%,
- la quantité de solide adsorbant en zone 3A est de 18%±8%,
- la quantité de solide adsorbant en zone 3B est de 14%±8%.
- la quantité de solide adsorbant en zone 4 est de 9%±8%.

### Etape B de fractionnement

Le procédé selon l'invention comprend une étape B de fractionnement par distillation dans au moins une première colonne 3 coupes de la fraction A2 contenant de l'éthylbenzène, de l'orthoxylène et du métaxylène et du désorbant issue de l'étape A de séparation. Ladite étape B permet l'obtention de
▪ une première fraction B2 contenant, de préférence constituée, de l'EB, de l'OX et du MX, et
▪ une seconde fraction B3 contenant, de l'OX et du MX et de l'EB, de préférence constituée, de l'OX et du MX et une quantité résiduelle en EB, et
▪ une fraction B42 contenant, de préférence constituée, du désorbant.

Avantageusement, la première fraction B2 et la seconde fraction B3 contiennent un mélange de EB, MX et OX dans des proportions variables de telle sorte que la teneur en EB dans les C8A de la première fraction est supérieure à celle de la seconde fraction. De préférence, l'écart de teneur en EB entre la première et la seconde fractions est supérieur à 1,0%, de préférence supérieur à 2,0%, de préférence supérieur à 2,5%, de préférence supérieure, à 3,0 %, et de préférence supérieur à 3,5. Avantageusement, la colonne 3 coupes mise en oeuvre à l'étape B présente un nombre de plateaux théoriques compris entre 30 et 80, de préférence entre 35 et 75, de manière préférée 40 et 70, de manière très préférée entre 45 et 65.

Selon l'invention, lorsque la seconde fraction de B3 contient du désorbant, ladite fraction est engagée à l'étape B de fractionnement dans une seconde colonne de distillation B-C4, permettant l'obtention d'une fraction B31 exempte de désorbant contenant du MX, et de l'OX, et une fraction B43 constituée de désorbant.

Avantageusement, lorsque le fractionnement du raffinat est réalisé dans une seule colonne 3 coupes à 60 plateaux théorique (mise en oeuvre ALT0) :
- La position de l'alimentation est située sur les plateaux 30 à 40 numérotés à partir du condenseur, et préférentiellement sur le plateau 37.
- La position du soutirage est située au moins 10 à 25 plateaux au-dessus de l'alimentation et de préférence 18 plateaux au-dessus de l'alimentation.

Avantageusement, lorsque le fractionnement du raffinat est réalisé dans une première colonne 3 coupes suivi d'une seconde colonne 2 coupes de préférence chacune à 47 plateaux théorique (mise en oeuvre ALT1, ALT2) :
- La position de l'alimentation sont situées sur les plateaux 16 à 24 numérotés à partir du condenseur, et préférentiellement entre les plateaux 18 à 22.
- La position du soutirage de la colonne 3 coupes est située entre 5 et 10 plateaux en dessous de la position d'alimentation et de préférence entre 7 et 8 plateaux en dessous de l'alimentation.
- Les positions des soutirages et des alimentations des colonnes de raffinats pourront être ajustées de façon homothétique en fonction du nombre total de plateaux installé dans chacune des colonnes de raffinat.

De préférence, la fraction A1 issue de l'étape A contenant, et de préférence constituée, d'un mélange de PX et de désorbant est engagée dans une étape de fractionnement par distillation dans une colonne de distillation (B-C1) permettant l'obtention d'une fraction B1 constituée de PX et une fraction B41 constituée de désorbant. Ladite distillation est mise en oeuvre selon les connaissances de l'Homme du métier.

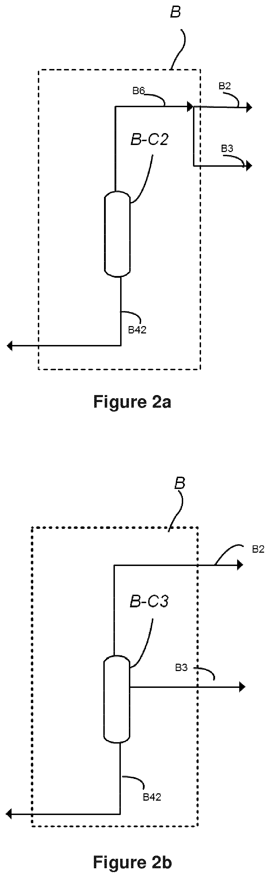

Dans un mode de réalisation descriptif (figure 2b, ALT0), la fraction A2 issue de l'étape A est engagée dans une étape de fractionnement par distillation dans une première colonne de distillation 3 coupes B-C3 permettant l'obtention en tête d'une fraction B2 contenant de l'EB, du MX et de l'OX, en soutirage latéral une fraction B3 exempte de désorbant et contenant de l'OX, du MX, de préférence constituée, de MX et de l'OX et d'une quantité résiduelle de EB et en fond une fraction B42 comprenant, de préférence constituée de désorbant .

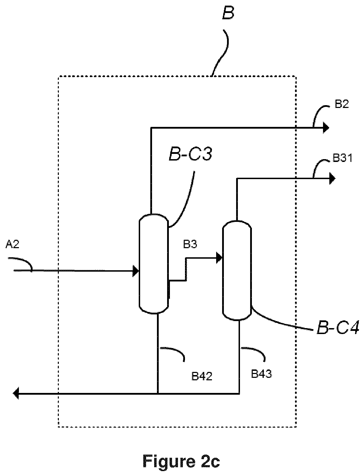

Dans un mode préféré de réalisation (figure 2c, ALT 1), ladite colonne 3 coupes B-C3 est mise en oeuvre de manière à obtenir
- en tête une première fraction B2 contenant, de préférence constituée, de l'EB, de l'OX et du MX,
- en soutirage latéral une seconde fraction B3 contenant de OX, du MX, de désorbant et d'une quantité résiduelle de EB, de préférence constituée d'OX, de MX et de désorbant, et,
- en fond une troisième fraction B42 comprenant, de préférence constituée de désorbant.

Ladite fraction B3 est engagée dans une seconde colonne de distillation 2 coupes B-C4 permettant l'obtention d'une fraction B31 exempte de désorbant contenant du MX, et de l'OX, et une fraction B43 constituée de désorbant.

Avantageusement, le mode de réalisation ALT1 (figure 2c) permet de diminuer la teneur en xylène dans la fraction B2 enrichie en EB.

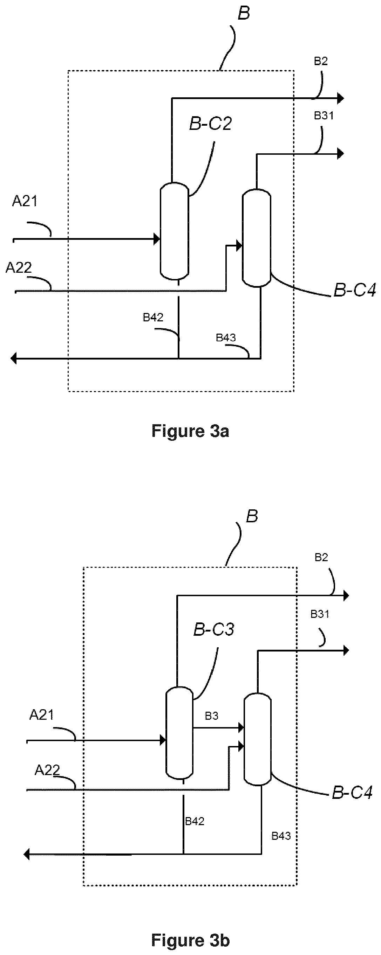

Dans le mode ALT2 selon l'invention, l'étape A permet l'obtention de deux fractions A21 et A22 (figure 3b, ALT 2). Ladite fraction A21 est engagée dans l'étape B de fractionnement par distillation dans une première colonne de distillation 3 coupes B-C3 permettant l'obtention
- en tête d'une première fraction B2 contenant, de préférence constituée, d'EB, d'OX et du MX,
- en soutirage latéral d'une seconde fraction B3 contenant de l'OX, du MX, de désorbant, et éventuellement une quantité résiduelle de EB, de préférence constituée d'OX, de MX et de désorbant, et
- en fond une troisième fraction B42 comprenant, de préférence constituée de désorbant.

Dans le mode de réalisation ALT2, ladite seconde fraction B3 issue de la première colonne mise en oeuvre à l'étape B et la fraction A22 issue de l'étape A sont engagées dans une seconde colonne de distillation B-C4 permettant l'obtention d'une fraction B31 contenant du MX, de l'OX, et éventuellement une quantité résiduelle de EB, de préférence constituée d'OX et du MX, et une fraction B43 comprenant, de préférence constituée de désorbant, ou la fraction A22 est introduite dans la seconde colonne, de préférence 2 coupes, de raffinat B-C4, en dessous du point d'injection de la fraction B3.

Avantageusement, dans ledit mode de réalisation ALT2 (figure 3b), la colonne en 3 coupes B-C3 ne reçoit que la fraction A21 enrichie en EB du raffinat A2, issue de l'étape A de séparation par SMB ce qui permet encore de diminuer la teneur en xylènes dans la fraction B2 enrichie en EB par rapport au mode de réalisation ALT1.

Avantageusement, les fractions désorbant B41, B42 et B43 exemptes de C8A sont récupérées en fond de chaque colonne de distillation assemblées en une fraction B4 et renvoyées vers l'étape A d'adsorption en lit mobile simulé.

Lorsque le désorbant de ladite étape A est un composé plus lourd que les xylènes, c'est-à-dire avec un point d'ébullition supérieur à celui des xylènes, le raffinat enrichi en EB est produit en tête de la colonne en 3 coupes, le raffinat enrichi en xylènes est obtenu dans le soutirage latéral, ou alternativement en tête de la seconde colonne de distillation.

Lorsque le désorbant de ladite étape de séparation est un composé plus léger que les xylènes, un point d'ébullition inférieur à celui des xylènes, le flux enrichi en Xylènes est produit en fond de la colonne 3 coupes, le raffinat enrichi en EB est obtenu dans le soutirage latéral, ou alternativement en fond de la seconde colonne de distillation, le désorbant exempt de C8A est soutiré en tête des deux colonnes et recyclé vers l'étape d'adsorption.

Avantageusement, les colonnes de distillation B-C1, B-C3 et B-C4 mises en oeuvre à l'étape B de fractionnement sont opérées à pression atmosphérique, la température de rebouillage est comprise entre 210 et 250 °C, de préférence comprise entre 220 et 240°C et de manière préférée est de 230°C, les taux de reflux exprimés en rapport massique reflux/charge sont compris entre 1,0 et 3,0 et préférentiellement entre 1,4 et 2,0.

Avantageusement le raffinat B2 récupéré en tête de la colonne B-C3 est obtenu sous forme d'un distillat liquide soutiré 4 plateaux sous le condenseur, ledit condenseur est à reflux total et comprend une botte de décantation pour soutirer de l'eau liquide.

Plus précisément, lorsque l'étape B met en oeuvre une colonne de distillation (B-C3) les performances de l'étape de fractionnement du raffinat obtenues par distillation (ALTO et ALT1) sont caractérisées par :
- le taux (TX) de récupération de l'EB dans le raffinat B2 enrichi en EB : TX(EB)= EB _{(raffinat B2)} / (EB _{(raffinat B2)} + EB _{(raffinat B3)})
- le taux (TX) de récupération des xylènes dans le raffinat B2 enrichi en EB : TX(XYL)= XYL _{(raffinat B2)} / (XYL _{(raffinat B2)} +XYL _{(raffinat B3)})

Lorsque l'étape B met en œuvre une seconde colonne de distillation (B-C4) les performances de l'étape de fractionnement du raffinat obtenues par distillation (ALT2) sont caractérisées par :
- le taux (TX) de récupération de l'EB dans le raffinat B2 enrichi en EB : TX(EB)= EB _{(raffinat B2)} / (EB _{(raffinat B2)} + EB _{(raffinat B31)})
- le taux (TX) de récupération des xylènes dans le raffinat B2 enrichi en EB : TX(XYL)= XYL _{(raffinat B2)} / (XYL _{(raffinat B2)} + XYL _{(raffinat B31)})

Avantageusement, dans le procédé selon l'invention, le taux de récupération en éthylbenzène (noté TX(EB)), est compris entre 50 % et 90 %, et de préférence entre 80% et 90 %.

Avantageusement, dans le procédé selon l'invention ATL 0 ou ATL 1, lorsque les fractions A1 et A2 sont engagées aux étapes de fractionnement, le taux récupération des xylènes TX(XYL) présente un écart d'au moins inférieur de 2 %, de préférence d'au moins 5 %, de préférence d'au moins 10 % et de manière préférée d'au moins 15 % par rapport taux de récupération de l'éthylbenzène (TX(EB)). Avantageusement, lorsque les fractions A1, A21 et A22 sont engagées aux étapes de fractionnement, le taux récupération des xylènes TX(XYL) présente un écart au moins strictement inférieur de 20 %, de préférence d'au moins 23 %.

Selon un autre mode de réalisation, l'étape de distillation peut être réalisée avec tout autre arrangement de distillation incluant une première colonne de distillation 3 coupes B-C3. De préférence, ladite colonne de distillation 3 coupes B-C3 des variantes ALT0, ALT1, ou ALT2 selon l'invention comprend une paroi interne, permettant d'améliorer les performances de séparation des deux raffinats B2, B3 exempts de désorbant.

### Etape C d'isomérisation en phase vapeur

Avantageusement le procédé comprend une étape C d'isomérisation en phase vapeur du raffinat B2 contenant de l'éthylbenzène, de l'orthoxylène et du métaxylène issu de l'étape B de fractionnement.

Avantageusement l'étape d'isomérisation en phase vapeur permet l'isomérisation de l'OX et du MX ainsi que de l'EB, dans une unité opérant en phase vapeur, à haute température et convertissant l'éthylbenzène en xylènes, pour traiter le raffinat B2 riche en EB issu de l'étape B.

De préférence, le rapport massique du raffinat B2 engagé à l'étape C d'isomérisation sur le raffinat total (B2 + B3 ou B2 + B31) obtenu à l'issu de l'étape B est compris entre 20 et 90%, préférentiellement entre 25% et 60% et plus préférentiellement entre 30 et 45%. Lesdits ratios permettent avantageusement de maximiser la production de paraxylène. Lorsque ledit ratio est élevé, il est possible d'augmenter la production de PX sans contrepartie sur la capacité de la boucle xylènes, lorsque ledit ratio est modéré la production de PX augmente plus sensiblement, mais s'accompagne d'une légère augmentation de capacité la boucle xylènes.

Un avantage du procédé selon l'invention est d'entraîner une augmentation de la concentration stationnaire de l'EB dans la boucle xylènes, ce qui permet une augmentation de la conversion en EB par passe dans la première unité d'isomérisation en phase vapeur.

Ainsi, la combinaison d'une étape de séparation par adsorption en SMB, à une étape B de fractionnement par distillation dans une colonne 3 coupes et de deux étapes isomérisations, permet d'améliorer le rendement global en paraxylène de la boucle aromatique et de minimiser l'impact économique.

Selon l'invention l'étape d'isomérisation en phase vapeur permet de convertir l'EB en xylènes avec un taux de conversion par passe de l'éthylbenzène généralement comprise entre 10% et 50%, de préférence comprise entre 20 et 40 %, avec une perte en C8 Aromatiques (C8A) inférieure à 5,0 %poids, de préférence inférieure à 3,0 % poids et préférentiellement inférieure à 1,8 % poids

L'étape d'isomérisation en phase vapeur est mise en oeuvre à une température supérieure à 300°C, de préférence comprise entre 350° et 480°C, une pression inférieure à 4,0 MPa, de préférence comprise entre 0,5 et 2,0 MPa, une vitesse spatiale inférieure à 10,0 h⁻¹, de préférence comprise entre 0,5 h⁻¹ et 6,0 h⁻¹, rapport molaire hydrogène sur hydrocarbure inférieur à 10,0, de préférence compris entre 3,0 et 6,0, et en présence d'un catalyseur comportant au moins une zéolithe présentant des canaux dont l'ouverture est définie par un anneau à 10 ou 12 atomes d'oxygène (10 MR ou 12 MR), et au moins un métal du groupe VIII de teneur comprise entre 0,1 et 0,3% poids.

Tous les catalyseurs susceptibles d'isomériser les hydrocarbures à 8 atomes de carbone, zéolithiques ou non, conviennent pour l'unité d'isomérisation en phase vapeur. De préférence, le catalyseur mis en oeuvre comprend une zéolithe acide, par exemple de type structural MFI, MOR, MAZ, FAU et/ou EUO. De manière encore plus préférée, le catalyseur mis en oeuvre comprend une zéolithe de type structural EUO et au moins un métal du groupe VIII de la classification périodique des éléments.

Selon une variante préférée du procédé, le catalyseur utilisé à l'étape C comprend de 1% à 70% poids d'une zéolithe de type structural EUO, de préférence EU-1, comprenant du silicium et au moins un élément T choisi de préférence parmi l'aluminium et le bore dont le rapport Si/T est compris entre 5 et 100.

De préférence, la zéolithe est sous forme hydrogène au moins en partie, et la teneur en sodium est telle que le ratio atomique Na/T est inférieur à 0,1.

De préférence, le catalyseur comprend entre 0,01 et 2% poids d'étain ou d'indium, et du soufre à raison de 0,5 à 2 atomes par atome de métal du groupe VIII.

L'effluent C1 obtenu à l'étape C, présentant des concentrations en isomère PX, OX et MX proches des concentrations de l'équilibre thermodynamique est recyclé vers l'étape A d'adsorption en lit mobile simulé.

Dans un mode de réalisation particulier, lorsque l'effluent C1 contient des composés lourds et légers formés par les réactions indésirables ledit effluent est alors engagé dans une étape optionnelle de fractionnement pour éliminer lesdits composés.

### Etape D d'isomérisation en phase liquide

Avantageusement le procédé comprend une étape D d'isomérisation en phase liquide de la fraction B3 ou de la fraction B31 contenant de l'orthoxylène et du métaxylène issue de l'étape B de fractionnement.

Le procédé selon l'invention permet ainsi d'augmenter la proportion de l'isomérisation des xylènes réalisée par le catalyseur opérant en phase liquide et conduisant aux plus faibles pertes en C8A. L'étape D d'isomérisation des xylènes est mise en oeuvre en phase liquide avec un taux de conversion de l'EB par passe inférieur ou égal à 5,0 %, de préférence inférieur ou égal à 3,0 %,et de préférence inférieur ou égale à 0,2 % et permet l'isomérisation du mélange de xylènes de telle sorte que le PX ait une approche à l'équilibre thermodynamique supérieure ou égale à 90,0 % et préférentiellement supérieure ou égale à 94,0%.

L'étape D d'isomérisation en phase liquide est mise en oeuvre à une température inférieure à 300°C, de préférence comprise entre 200 et 260°C, une pression inférieure à 4,0 MPa, de préférence comprise entre 1,0 et 3,0 MPa, une vitesse spatiale inférieure à 5,0 h⁻¹ et de préférence comprise entre 2,0 et 4,0 h-1, et en présence d'un catalyseur comportant au moins une zéolithe présentant des canaux dont l'ouverture est définie par un anneau à 10 ou 12 atomes d'oxygène (10 MR ou 12 MR), préférentiellement un catalyseur comportant au moins une zéolithe présentant des canaux dont l'ouverture est définie par un anneau à 10 atomes d'oxygène (10 MR), et de manière encore plus préférée un catalyseur comportant une zéolithe de type ZSM-5.

Le brevet US 8697929 décrit avec plus de précision les conditions, opératoires ainsi que les catalyseurs des isomérisations en phase vapeur et en phase liquide pouvant être mis en oeuvre dans le procédé selon l'invention.

Avantageusement, l'effluent D1 obtenu à l'étape D, présentant des concentrations en isomère PX, OX et MX proches des concentrations de l'équilibre thermodynamique sont recyclés vers l'étape A d'adsorption en lit mobile simulé.

Dans un mode de réalisation particulier, lorsque l'effluent D1 contient des composés lourds et légers formés par les réactions indésirables ledit effluent est alors engagé dans une étape optionnelle de fractionnement pour éliminer lesdits composés.

### EXEMPLES

Les exemples 2, 4-5 ci-dessous illustrent l'invention sans en limiter la portée.

### Exemple 1 (Référence) :

Cet exemple montre l'intérêt de l'invention en comparant les performances de l'étape de distillation B selon l'état de l'art Figure 2 a), le procédé non inclus dans l'invention Figure 2b) et 2c) respectivement dans ses modes de réalisation noté ALT0 et ALT1.

Dans cet exemple on considère 545 t/h d'une coupe C8 (2) issue d'une colonne de xylène et comprenant des C8Aromatiques (C8A) provenant d'un réformat, d'une unité de transalkylation et d'une ou plusieurs unités d'isomérisation, sa composition est en % poids :

| | |
|---|---|
| C8- | 0,4% |
| EB | 4,1% |
| PX | 23,0% |
| MX | 50,1% |
| OX | 22,3% |
| C9+ | 0,1% |

Les C8- correspondent aux composés comprenant moins de 8 atomes de carbones.
- Etape A de séparation en lit mobile simulé (SMB)

La coupe C8A est envoyée dans une unité A d'adsorption en lit mobile simulé comprenant 4 zones délimitées par les injections de charges et de désorbant (B4) et les soutirages de raffinat (A2) et d'extrait (A1) . L'étape de séparation est mise en oeuvre dans une unité d'adsorption en lit simulé composée de 15 lits contenant de la zéolithe X échangée au baryum répartis comme suit :
∘ 3 lit en Zone 1, comprise entre l'injection du désorbant B4 et le soutirage de l'extrait A1.
∘ 6 lits en zone 2, comprise entre le soutirage de l'extrait A1 et l'injection de la charge
∘ 4 lits en zone 3, comprise entre l'injection de la charge et le soutirage du raffinat A2
∘ 2 lits en zone 4, comprise entre le soutirage du raffinat A2 et l'injection du désorbant B4

La température est de 175 °C. Le désorbant utilisé est le Paradiéthylbenzène, le taux de désorbant par rapport à la charge est de 1,2 (vol/vol).

Ainsi mise en œuvre de l'étape A de séparation par adsorption permet de produire 2 fractions A1 et A2 alimentant l'étape B de distillation :
- Une fraction A1 contenant au moins 97,0 % du PX de la charge et une partie du désorbant, ladite fraction est envoyée à une étape de distillation dans une colonne d'extrait B-C1 afin de récupérer le PX pur en tête (flux B1) et l'adsorbant en fond (flux B41).
- 829 t/h d'un raffinat A2 appauvri en PX, contenant 407 t/h de désorbant

### • Mise en oeuvre de l'étape B de fractionnement par distillation selon art antérieur (figure 2a)

Dans ce mode de réalisation, le raffinat (A2) est alimenté au plateau théorique 24 dans la colonne de distillation (B-C2) contenant 47 plateaux théoriques, un condenseur et un rebouilleur, opérée à 0,2 MPa avec un taux de reflux de 1,3. Ladite colonne permet de produire 2 flux :
- 422 t/h d'un raffinat en tête (B6) exempt de désorbant et
- 407 t/h de désorbant (B42) en fond exempt de C8A et renvoyé à l'étape A, après une étape d'échange thermique à la température requise pour l'adsorption.

Le raffinat (B6) est fractionné en 2 flux. 90,0 % du raffinat (B6) exempt de désorbant, noté (B2), est envoyé vers la première étape d'isomérisation (C), et 10% du raffinat résiduel (B6) noté (B3) est alimenté vers la deuxième étape d'isomérisation (D).

Ledit fractionnement du raffinat (A2) par la colonne de distillation nécessite 86,6 Gcal/h d'énergie de rebouillage.

### • Mise en oeuvre de l'étape B de fractionnement par distillation selon invention

Dans un premier mode de réalisation (figure 2b, ALT0) selon l'invention, le raffinat (A2) subit une étape B de séparation. Ledit raffinat est alimenté au plateau théorique 37 dans la colonne de distillation en 3 coupes (B-C3) contenant 60 plateaux théoriques, un condenseur et un rebouilleur, opérée à 0,2 MPa avec un taux de reflux de 1,9. Ladite colonne permet de produire 3 flux :
- 367 t/h d'un raffinat en tête (B2) contenant 90,0 % de l'éthylbenzène et 87,0 % d'un mélange constitué de OX et MX contenu dans le raffinat A2.
- 54 t/h d'un raffinat (B3) obtenu en soutirage latéral sur le plateau théorique 19 exempt de désorbant et
- en fond 407 t/h du désorbant (B42) exempt de C8A et renvoyé à l'étape A, après une étape d'échange thermique à la température requise pour l'adsorption.

Ledit fractionnement du raffinat A2 par la colonne de distillation 3 coupes nécessite 86 Gcal/h d'énergie de rebouillage.

Dans un second mode de réalisation préféré (figure 2c, ALT1) selon l'invention, le raffinat (A2) subit une étape B de séparation. Ledit raffinat est alimenté au plateau théorique 22 dans la colonne de distillation 3 coupes (B-C3) contenant 47 plateaux théoriques, un condenseur et un rebouilleur, opérée à 0,2 MPa avec un taux de reflux de 1,47. Cette colonne permet de produire 3 flux :
- 348 t/h d'un raffinat en tête (B2) contenant 90,0 % de l'éthylbenzène contenu dans le raffinat total (B2+B31), et 82,0 % % des xylènes contenu dans le raffinat total (B2+B31).
- 202 t/h d'un raffinat (B3) obtenu en soutirage latéral sur le plateau théorique 30 et
- en fond 278 t/h de désorbant (B42) exempt de C8A et renvoyé à l'étape A, après une étape d'échange thermique à la température requise pour l'adsorption.

Le raffinat (B3) contenant du désorbant est envoyé sur le plateau 22 de la seconde colonne de distillation (B-C4) contenant 47 plateaux théoriques un condenseur et un rebouilleur, opérée à 0,2 MPa et avec un taux de reflux de 2,1 %, qui permet de récupérer en tête 73 t/h d'un raffinat B31 constitué par du MX,OX, appauvri en PX et avec une teneur en EB de 3,0 % poids et enfin 129 t/h d'un produit de fond (B43) contenant du désorbant exempt de C8A et renvoyé à l'étape A, après une étape d'échange thermique à la température requise pour l'adsorption. Le raffinat B31 est envoyé à l'étape d'isomérisation D opérant en phase liquide. Le fractionnement du raffinat selon cette mise en oeuvre ALT1 nécessite 86,1 Gcal/h d'énergie de rebouillage.

A l'issue de l'étape de fractionnement du raffinat A2 dans ces différentes mises en œuvre selon l'invention, le raffinat B2 riche en EB est envoyé dans l'étape d'isomérisation C, le raffinat B31 est envoyé dans l'étape d'isomérisation D.

Les performances de l'étape de séparation du raffinat selon l'état de l'art FIG 2a), ou selon l'invention FIG2b), et FIG2c) sont résumées ci-après :

| | Art antérieur | Selon invention ALT0 | Selon invention ALT1 |
|---|---|---|---|
| | Figure 2a) | Figure 2b) | Figure2c) |
| TX(EB) | 90,0% | 90,0% | 90,0% |
| TX(XYL) | 90,0% | 87,0% | 82,2% |
| Total Qreb (Gcal/h) | 86,6 | 86,0 | 86,1 |
| Nb plat-Col B-C2 | 47 | - | - |
| Nb plat-Col B-C3 (3 coupes) | - | 60 | 47 |
| Nb plat-Col B-C4 | - | - | 47 |

Cet exemple illustre l'intérêt de de la demande lorsque l'étape d'adsorption produit un raffinat. Elle permet pour un même taux de récupération de l'EB de 90,0 % dans le raffinat B2 de réduire de 3 points ou de 8 points le taux de récupération des xylènes associé selon la mise en oeuvre de l'invention. Par rapport à l'état de l'art, dans lequel les taux de récupération sont identiques, cet enrichissement permet d'augmenter la proportion du raffinat alimentant l'unité d'isomérisation en phase liquide présentant des pertes en C8A modérée et de réduire celle alimentant l'unité d'isomérisation en phase gaz présentant des pertes en C8A supérieures. Par suite, le rendement en PX du complexe aromatique augmente comme illustré dans l'exemple 3.

Cet enrichissement nécessite de mettre en oeuvre au moins une première colonne de distillation 3 coupes , sans impact sur l'énergie requise au rebouillage de l'étape B de fractionnement, ni sur la configuration et l'opération de l'étape A d'adsorption

Par rapport à l'état de l'art il est possible d'augmenter le rendement en PX du complexe aromatique, comme illustré dans l'exemple 3.

### Exemple 2 :

Cet exemple montre l'intérêt de l'invention en comparant les performances de l'étape de distillation B selon l'état de l'art (Figure 3 a)), avec le procédé selon l'invention (Figure 3b)) dans un mode de réalisation appelé ALT2.

### • Etape A de séparation en lit mobile simulé (SMB)

Dans cet exemple on considère 545 t/h d'une coupe C8 (2) issue d'une colonne de xylène et comprenant des C8 Aromatiques provenant d'un réformat, d'une unité de transalkylation et d'une ou plusieurs unité d'isomérisation. La composition de ladite coupe est donnée en % poids :

| | |
|---|---|
| C8- | 0,4% |
| EB | 4,1% |
| PX | 23,0% |
| MX | 50,1% |
| OX | 22,3% |
| C9+ | 0,1% |

Les C8- correspondent aux composés comprenant moins de 8 atomes de carbones.

Cette coupe C8A est envoyée dans une unité A d'adsorption en lit mobile simulé comprenant 5 zones délimitées par les injections de charges et de désorbant (B4) et les soutirages des raffinat (A21, A22) et d'extrait (A1). Ladite unité d'adsorption en lit simulé est composée de 18 lits contenant de la zéolithe X échangée au baryum répartis comme suit :
∘ 3 lit en Zone 1, comprise entre l'injection du désorbant (B4) et le soutirage de l'extrait (A1).
∘ 6 lits en zone 2, comprise entre le soutirage de l'extrait (A1) et l'injection de la charge,
∘ 4 lits en zone 3A, comprise entre l'injection de la charge et le soutirage du raffinat (A21),
∘ 3 lits en zone 3B, comprise entre le soutirage du raffinat (A21) et le soutirage du raffinat (A22),
∘ 2 lits en zone 4, comprise entre le soutirage du raffinat (A22) et l'injection du désorbant (B4)

La température est de 175 °C, le désorbant utilisé est le Paradiethylbenzene, le taux de désorbant par rapport à la charge est de 1,2 (vol/vol).

Ainsi la mise en oeuvre d'une étape A de séparation en SMB permet l'obtention 3 flux notés (A1), (A21) et (A22) alimentant l'étape de distillation B. Les trois flux présentent les caractéristiques suivantes :
- Un extrait (A1) contenant au moins 97,0 % du paraxylène PX de la charge et une partie du désorbant, lequel est envoyé dans une colonne d'extrait afin de récupérer le PX pur en tête et le désorbant en fond.
- 508 t/h d'un premier raffinat (A21) appauvri en de PX, riche en éthylbenzène, contenant 98,0 % de EB de l'éthylbenzène contenu dans le raffinat total B2+B31et 78,0 % des xylènes contenu dans le raffinat total B2+B31
- 320 t/h d'un second raffinat (A22) appauvri en PX, qui contient essentiellement un mélange MX et OX, dont la teneur en EB dans la coupe A8 est de 0,5%, et une autre partie du désorbant.

### • Mise en oeuvre de l'étape B de fractionnement par distillation selon art antérieur (figure 3a)

Dans ce mode de réalisation, le premier raffinat (A21) contenant du désorbant est alimenté au plateau théorique 27 dans la colonne de distillation (B-C2) contenant 47 plateaux théoriques, un condenseur et un rebouilleur, opérée à 0,2 MPa avec un taux de reflux de 1,1. Le premier raffinat sans désorbant (B2) est récupéré en tête et est envoyé à la première étape d'isomérisation opérant en phase vapeur. Le deuxième raffinat contenant du désorbant (A22) est engagé dans une étape B de séparation. Ledit raffinat est alimenté au plateau théorique 21 dans la colonne de (B-C4) contenant 47 plateaux théoriques, un condenseur et un rebouilleur, opérée à 0,2 MPa et avec un taux de reflux de 2,7. Le deuxième raffinat sans désorbant B31 est récupéré dans le distillat et est envoyé à l'étape D d'isomérisation opérant en phase liquide. Les deux flux de désorbant B42 et B43 exempts de C8A, issus du fond des deux colonnes de raffinat (B-C2 et B-C4) sont mélangés et renvoyés vers l'étape de séparation en lit mobile simulé, après une étape d'échange thermique à la température requise pour l'adsorption.

L'étape B de fractionnement des deux raffinats (A21) et (A22) produits par le lit mobile simulé nécessite 81,2 Gcal/h d'énergie de rebouillage.

### • Mise en oeuvre de l'étape B de fractionnement par distillation selon invention (figure 3b, ALT2)

Dans un mode de réalisation (ALT2) selon l'invention, le premier raffinat (A21) contenant du désorbant est alimenté au plateau théorique 20 dans la colonne de distillation en 3 coupes (B-C3) contenant 47 plateaux théoriques, un condenseur et un rebouilleur, opérée à 0,2 MPa avec un taux de reflux de 1,38. Ladite colonne permet de produire 3 flux :
- 284 t/h d'un premier raffinat (B2) en tête contenant 90,1% de l'éthylbenzène contenu du raffinat total B2+B31 ,
- 73,6 t/h d'une deuxième coupe (B3) soutirée en phase liquide du plateau théorique 27, contenant l'éthylbenzène résiduel et 12% des xylènes du raffinat total B2+B31 avec du désorbant, et
- 150,6 t/h d'un produit de fond B42 contenant du désorbant exempt de composés en C8A.

Le raffinat (B3) contenant du désorbant est introduit sur le plateau 18 d'une seconde colonne de distillation (B-C4) contenant 47 plateaux théoriques un condenseur et un rebouilleur, opérée à 0,2 MPa et avec un taux de reflux de 2,1.

Le second raffinat (A22) issu de l'étape A de séparation en lit mobile simulé est engagé dans l'étape B de distillation. Ledit raffinat (A22) est introduit sur le plateau 24 de cette colonne de distillation (B-C4). 137 t/h d'un raffinat (B31) sont récupérés en tête . Ledit raffinat (B31) est sans désorbant et constitué de MX, OX, appauvri en PX et avec une teneur en EB de 1,6 % poids. Le raffinat (B31) est envoyé à l'étape D d'isomérisation opérant en phase liquide. Les deux flux de désorbant B42 et B43 issus des deux colonnes de raffinat (B-C3 et B-C4) sont mélangés et renvoyés au lit mobile simulé, après échange thermique à la température requise pour l'adsorption.

Le fractionnement des deux raffinats (A21) et (A22) nécessite 84,5 Gcal/h d'énergie de rebouillage. Les performances de l'étape de séparation B du raffinat selon l'état de l'art (figure 3a), ou selon l'invention (figure 3b) sont résumées ci-après :

| | **Art Antérieur** | **Invention ALT2** |
|---|---|---|
| | **figure 3a****)** | **figure 3b****)** |
| TX(EB) | 98,0% | 90,2% |
| TX(XYL) | 78,0% | 66,1 % |
| Total Qreb (Gcal/h) | 81,2 | 84,5 |
| Nb plat-raf col B-C2 | 47 | - |
| Nb plat-raf col B-C3 (3 coupes) | - | 47 |
| Nb plat-raf col2 B-C4 | 47 | 47 |

Cet exemple illustre l'intérêt de l'invention lorsque l'étape d'adsorption A produit 2 raffinats. Elle permet de réaliser par le couplage d'une étape de distillation et d'adsorption une meilleure séparation EB/XYL que la séparation décrite dans l'état de l'art, sans toutefois impacter le nombre d'équipements ni l'énergie requise pour le rebouillage

L'étape B de séparation selon l'invention nécessite de mettre en oeuvre une première colonne de distillation 3 coupes couplée à une seconde colonne de distillation.

Par rapport à l'état de l'art il est possible d'augmenter le rendement en PX du complexe aromatique, comme illustré dans l'exemple 4.

### Exemple 3 (Référence) :

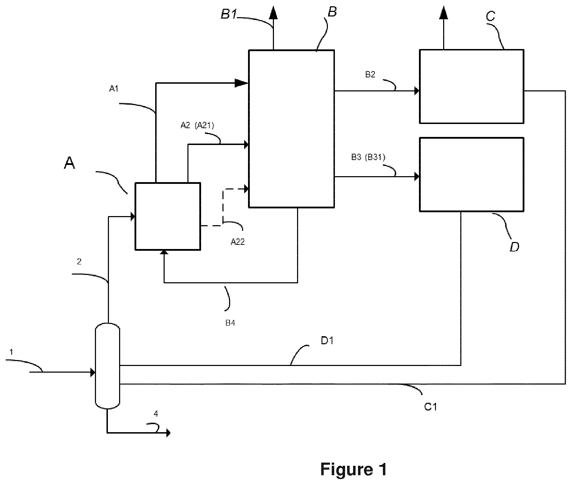

Cet exemple montre l'intérêt de la demande en détaillant les performances d'une boucle xylène représentée sur la figure 1 comprenant
- une étape d'adsorption A produisant uniquement 1 raffinat A2,
- une étape B de fractionnement du raffinat, et
- deux étapes d'isomérisation C et D.

Pour bien comprendre l'intérêt de l'invention, l'étape de séparation B est effectuée selon l'état de l'art, ou selon les versions ALT0 et ALT1 telles que décrites dans l'exemple 1.

Le taux de récupération de l'EB, noté TX(EB), est fixé à 90,0 % et les taux de récupération des xylènes, noté TX(XYL), varient entre 90,0 %, 87,0 % et 82,0 %.

Ladite boucle xylènes est alimentée par un débit de 100 t/h d'une coupe C8+ (flux 1, figure 1) provenant d'un reformat contenant 68 t/h de coupe C8A dont la composition massique est donnée ci-dessous :

| | |
|---|---|
| EB | 16,7% |
| PX | 18,0% |
| MX | 41,0% |
| OX | 24,3% |

Ladite coupe C8A (flux 1, figure 1) est introduite, avec les recycles des effluents (C1, D1) des deux étapes C et D d'isomérisation, au plateau théorique 15 dans la colonne de xylènes contenant 72 plateaux, opérée avec un taux de reflux de 1,8, permettant de récupérer en tête 99,8 % des C8A dans le distillat et d'éliminer les C9 + de la charge (flux 2, figure 1) de l'unité de séparation du PX en lit mobile simulé.

Ladite coupe C8A est engagée dans une étape A de séparation en lit mobile simulé produisant un extrait A1 riche en PX et en désorbant et un raffinat A2 appauvri en PX et contenant du désorbant. L'extrait A1 et le raffinat A2 sont envoyés dans une étape de fractionnement B tel que décrite dans l'exemple 1, comportant plusieurs colonnes de distillation. Elle permet de recycler le désorbant vers l'étape A d'adsorption, de produire du PX avec une pureté de 99,8%, et deux raffinats l'un enrichi en EB (B2) et l'autre appauvri en EB (B3 ou B31) tels que décrit dans l'exemple 1 et illustrés sur les figures 2a, 2b, 2c.

Le raffinat (B2), mélangé à un recyclage d'hydrogène, est alimenté à l'unité de conversion de l'éthylbenzène dans la première étape d'isomérisation C, travaillant dans les conditions suivantes : Pression : 0,9 MPa, Température: 376°C, rapport H₂/HC= 4,1 Catalyseur : contient du platine et de la zéolithe EU-1 vitesse spatiale: 5 h⁻¹.

Dans ces conditions la conversion de l'EB est de 28,9 % (état de l'art) ; 29,3% (selon invention versionALT0) ; 29,9 %(selon invention versionALT1) les pertes en C8A sont de 1,8 % poids et l'approche à l'équilibre du PX est de 92,0 % et du OX de 87,0%

En sortie du réacteur, un train de séparation permet de produire un gaz contenant majoritairement de l'hydrogène (C2), du toluène et une coupe paraffinique et naphténique renvoyée à l'entrée du réacteur. La partie la plus lourde de l'effluent (C1), est recyclée vers l'entrée de la colonne de xylènes (figure 1).

Le raffinat B3 est alimenté à l'unité d'isomérisation des xylènes dans la deuxième étape D d'isomérisation, travaillant dans les conditions suivantes : température 240°C, pression 1,8 MPa, vitesse spatiale 2,5 h⁻¹, avec un catalyseur contenant une zéolithe de type ZSM-5.

Dans ces conditions la conversion de l'EB est de 3,4 % et l'approche à l'équilibre du PX est de 94,5 %.

L'effluent de la section réactionnelle (D1) est recyclé à la colonne de xylènes (figure 1).

Le bilan matière de la boucle xylènes ainsi décrite est résumé dans le tableau ci-dessous :

| | Art antérieur 1 | Invention ALT0 | Invention ALT1 |
|---|---|---|---|
| nombre d'unité d'isomérisation | 2 | 2 | 2 |
| nombre d'effluent de l'unité d'adsorption | 2 | 2 | 2 |
| Fractionnement du raffinat par une colonne de distillation | 2 coupes | 3 coupes | 3 coupes + 1 side colonne |
| Performance de l'étape B de fractionnement TX(EB) | 0,90 | 0,90 | 0,90 |
| TX(XYL) | 0,90 | 0,87 | 0,82 |
| Capacité des unités (kta) | | | |
| Etape A | 2917 | 2912 | 2904 |
| Etape C | 2142 | 2080 | 1981 |
| Etape D | 237 | 294 | 383 |
| débit total de raffinat sans désorbant | 2379 | 2373 | 2364 |
| fraction du raffinat alimenté à la première étape d'isomérisation (C) | 90,0% | 87,6% | 83,8% |
| Tamis et catalyseurs (t) | | | |
| quantité de tamis d'adsorption (Etape A) | 476,0 | 475,7 | 475,2 |
| quantité de catalyseur (Etape C) | 51 | 50 | 47 |
| quantité de catalyseur (Etape D) | 11 | 14 | 18 |
| Production | | | |
| PX produit (kt/y) | 527,7 | 528,5 | 529,9 |
| Delta Gain produit (MM$/y) | 0 | 1,27 | 3,29 |

Cet exemple illustre une augmentation de la productivité d'une boucle xylènes contenant 2 isomérisations, avec un lit mobile simulé produisant 2 effluents en utilisant une première colonne de distillation 3 coupes dans l'étape de fractionnement B.

La consommation énergétique de l'étape de fractionnement B n'est pas impactée. Le gain de production de PX, estimé en se basant sur un prix moyen de PX de 1500$/t ($/tonne) est de 1,3 MM$/y (millions de dollars / an) pour la version ALT0 et 3,3 MM$/y pour la version ALT1 et justifient l'investissement marginal associé aux modifications de l'étape de fractionnement B.

### Exemple 4 :

Cet exemple montre l'intérêt de l'invention en détaillant les performances d'une boucle xylènes représentée sur la figure 1 comprenant une étape d'adsorption A produisant 2 raffinats A21 et A22, une étape B de fractionnement du raffinat, et deux étapes d'isomérisation C et D. Pour illustrer l'intérêt de l'invention l'étape de séparation B est effectuée selon l'état de l'art, ou selon la version ALT2 décrite dans l'exemple 2. Ainsi les taux de récupération en EB TX(EB), et en xylènes TX(XYL), obtenus par le procédé selon l'état de l'art sont respectivement de 98,0 et 78,0%, alors que selon la version ALT2 du procédé selon l'invention, les taux de récupération sont respectivement de 90,0 % et 66, %.

La boucle xylènes est alimentée par 100 t/h d'une coupe C8+ (flux 1, figure 1) provenant d'un reformat contenant 68 t/h de coupe C8A dont la composition massique est donnée ci-dessous :

| |
|---|
| EB 16,7% |
| PX 18,0% |
| MX 41,0% |
| OX 24,3% |

Ladite coupe C8A (flux 1, figure 1) est introduite, avec les recycles des effluents des deux étapes d'isomérisation, au plateau théorique 15 dans la colonne de xylènes contenant 72 plateaux, opérée avec un taux de reflux de 1,8, permettant de récupérer en tête 99,8 % des C8Aromatiques (C8A) dans le distillat et d'éliminer les C9 + de la charge (flux 2, figure 1) de l'unité de séparation du PX en lit mobile simulé. Ladite coupe C8A est alimentée dans un lit mobile simulé produisant un extrait A1 riche en PX et en désorbant et deux raffinats A21 et A22 appauvri en PX et contenant du désorbant.

L'extrait et le raffinat sont envoyés dans une étape de séparation B, décrite dans l'exemple 1, comportant plusieurs colonnes de fractionnement. Le désorbant (B4) ainsi séparé est recyclé vers l'étape A d'adsorption. Ladite étape B permet de produire du Paraxylène avec une pureté de 99,8%, et deux raffinats enrichis en EB (B2) ou appauvri en EB (B3 ou B31) tels que décrit dans l'exemple 2 et illustré sur les figures 3a et 3b.

Le raffinat B2, mélangé à un recyclage d'hydrogène, est alimenté à l'unité de conversion de l'éthylbenzène dans la première étape d'isomérisation C, travaillant dans les conditions suivantes : Pression : 0,9 MPa, Température: 376°C, rapport H2/HC= 4:1 Catalyseur: contient du platine et de la zéolithe EU-1 vitesse spatiale: 5 h-1.

Dans ces conditions la conversion de l'EB est de 30,6 % (état de l'art); 32,1% (invention version ALT2), et les pertes en C8A sont de 1,8 % poids et l'approche à l'équilibre du PX est de 92 % et du OX est de 87%

En sortie du réacteur, un train de séparation permet de produire un gaz contenant majoritairement de l'hydrogène C2, du toluène et une coupe paraffinique et naphténique renvoyée à l'entrée du réacteur. La partie la plus lourde de l'isomérat (C1), est recyclée vers l'entrée de la colonne de xylènes.

Le raffinat B31 est alimenté à l'unité d'isomérisation des xylènes dans la deuxième étape d'isomérisation D, travaillant dans les conditions suivantes : température 240°C, pression 1,8 MPa, vitesse spatiale 2,5 h-1, avec un catalyseur contenant une zéolithe de type ZSM-5.

Dans ces conditions la conversion de l'EB est de 3,4 et l'approche à l'équilibre du PX est de 94,5%. L'effluent de la section réactionnelle (D1) est recyclé à la colonne de xylène.

Le bilan matière de la boucle xylènes est résumé dans le tableau ci-dessous :

| | **Art antérieur 2** | **Invention ALT2** |
|---|---|---|
| nombre d'unité d'isomérisation | 2 | 2 |
| nombre d'effluent de l'unité d'adsorption | 3 | 3 |
| Fractionnement du raffinat | par adsorption + 2 colonnes de raffinat | par adsorption + 1 col de raffinat 3 coupes + 1 side colonne |
| Performance de l'étape B de fractionnement | | |
| TX(EB) | 0,98 | 0,90 |
| TX(XYL) | 0,78 | 0,66 |

### Capacité des unités (kta)

| | | |
|---|---|---|
| Etape A | 2866 | 2878 |
| Etape C | 1897 | 1662 |
| Etape D | 428 | 672 |
| Débit total de raffinat sans désorbant | 2325 | 2334 |
| fraction du raffinat alimenté à la première étape d'isomérisation C | 81,6% | 71,2% |

### Tamis et catalyseurs (t)

| | | |
|---|---|---|
| quantité de tamis d'adsorption (Etape A) | 565,1 | 567,8 |
| quantité de catalyseur (Etape C) | 45 | 40 |
| quantité de catalyseur (Etape D) | 20 | 32 |

### Production

| | | |
|---|---|---|
| PX produit (kt/y) | 531,9 | 534,2 |
| Delta Gain produit (MM$/y) | 0 | 3,4 |

Ces résultats illustrent clairement que le procédé selon l'invention permet d'augmenter le rendement en Paraxylène d'une boucle xylène par la mise en oeuvre d'un lit mobile simulé produisant 3 effluents, en utilisant une première colonne de distillation 3 coupes dans l'étape de séparation B comme décrit dans l'exemple 1. Avantageusement cette mise en oeuvre particulier du procédé selon l'invention n'impacte pas le nombre d'équipement et la dépense énergétique.

Le gain estimé en se basant sur un prix moyen de PX de 1500$/t est de 3,4 MM$/y.

### Exemple 5 :

Dans une version particulière appelée ALT3, le procédé est mis en oeuvre avec les même équipements et selon la même configuration de la boucle xylènes que pour la version ALT2 de l'exemple 4 (figure 3b). Cet exemple illustre l'intérêt de l'invention lorsqu'on souhaite obtenir de très hauts rendements en PX, à partir de la même charge, moyennant une augmentation marginale de catalyseur d'isomérisation et de tamis.

Les paramètres opératoires de l'étape A d'adsorption sont identiques à ceux de l'exemple 4, en revanche, les paramètres opératoires de l'étape B de fractionnement du raffinat sont modifiés de telle sorte que le débit de raffinat B2 riche en EB de la 1ere colonne de distillation B-C3 est diminué au profit du débit de soutirage latéral B3 alimentant la seconde colonne de distillation B-C4 ainsi que son débit de distillat (B31) résultant.

Dans ce mode de réalisation les nouvelles performances de l'étape B de fractionnement sont telles que TX(EB)= 60% et TX (XYL)=38 %. Ledit raffinat B2 est ensuite engagé dans l'étape C d'isomérisation. Cette mise oeuvre permet d'augmenter la proportion de l'isomérisation des xylènes par le catalyseur présentant le moins de pertes en C8A, et in fine d'augmenter le rendement en PX.

La conversion par passe de l'EB à l'étape D d'isomérisation étant faible, le taux de recyclage de l'EB augmente ce qui a comme effet d'augmenter sa concentration stationnaire. Par suite la conversion par passe de l'EB dans la première isomérisation en phase vapeur, gouvernée par un équilibre thermodynamique est aussi augmentée et atteint 36,5 %.

Avantageusement la combinaison des deux étapes d'isomérisation selon l'invention permet d'atteindre de forte production de PX, moyennant de faibles contreparties d'augmentation de capacité.

Le bilan matière de la boucle xylènes ainsi décrite est résumé dans le tableau ci-dessous :

| | **Art antérieur 2** | **Invention ALT3** |
|---|---|---|
| nombre d'unité d'isomérisation | 2 | 2 |
| nombre d'effluent de l'unité d'adsorption | 3 | 3 |
| Fractionnement du raffinat | par adsorption + 2 colonnes de raffinat | par adsorption + 1 col de raffinat 3 coupes + 1 side colonne |
| Performance de l'étape B de fractionnement | | |
| TX(EB) | 0,98 | 0,60 |
| TX(XYL) | 0,78 | 0,38 |

### Capacité des unité (kta)

| | | |
|---|---|---|
| Etape A | 2866 | 2969 |
| Etape C | 1897 | 1063 |
| Etape D | 428 | 1357 |
| débit total de raffinat sans désorbant | 2325 | 2419 |
| fraction du raffinat alimenté à la première étape d'isomérisation C | 81,6% | 43,9% |

### Tamis et catalyseurs (t)

| | | |
|---|---|---|
| quantité de tamis d'adsorption (Etape A) | 565,1 | 586 |
| quantité de catalyseur (Etape C) | 45 | 25 |
| quantité de catalyseur (Etape D) | 20 | 64 |

### Production

| | | |
|---|---|---|
| PX produit (kt/y) | 531,9 | 537,9 |
| Delta Gain produit (MM$/y) | ,0 | 9,0 |

## Revendications

1. Procédé de production de paraxylène à partir d'une charge contenant des xylènes, de l'éthylbenzène et des hydrocarbures en C9+, comprenant
- une unique étape A de séparation en lit mobile simulé de ladite charge mise en oeuvre avec une zéolithe comme adsorbant et un désorbant, à une température comprise entre 20 et 250°C, sous une pression comprise entre 1,0 et 2,2 MPa, et avec un rapport volumique du désorbant sur la charge dans l'unité de séparation en lit mobile simulé est compris entre 0,4 et 2,5 ; ladite étape A permettant l'obtention de
▪ une première fraction (A1) contenant un mélange de paraxylène et de désorbant, et
▪ d'au moins une deuxième fraction (A2 ou A21) contenant de l'éthylbenzène (EB), de l'orthoxylène (OX) et du métaxylène (MX) et du désorbant,
- une étape B de fractionnement par distillation dans au moins une première colonne de distillation (B_C3) de ladite deuxième fraction issue de l'étape A permettant l'obtention de 3 fractions :
▪ une première fraction (B2) contenant de l'EB, de l'OX et du MX,
▪ une seconde fraction (B3) contenant de l'OX et du MX, et
▪ une troisième fraction (B42) contenant du désorbant,
dans lequel
l'étape A de séparation permet l'obtention d'une troisième fraction A22 appauvri en EB contenant un mélange du MX, de l'OX et du désorbant, lesdites fractions A21 et A22 étant envoyées dans ladite étape B de fractionnement,
la troisième fraction (A22) issue de l'étape A est engagée dans une seconde colonne de distillation (B-C4) permettant l'obtention d'une fraction (B31) exempte de désorbant contenant du MX et de l'OX, et d'une fraction B43 constituée de désorbant,
la seconde fraction de (B3) est engagée à l'étape B de fractionnement dans une seconde colonne de distillation (B-C4), permettant l'obtention d'une fraction (B31) exempte de désorbant contenant du MX, et de l'OX, et une fraction B43 constituée de désorbant,
la troisième fraction (A22) issue de l'étape A est introduite dans la seconde colonne de distillation (B-C4) en dessous du point d'injection latéral de la seconde fraction (B3) issue de la première colonne de distillation (B-C3).

2. Procédé selon la revendication 1 dans lequel la première fraction (B2) issue de l'étape B présente une teneur en EB supérieure à la teneur en EB de la seconde fraction (B3) issue de la première colonne (B_C3)mise en oeuvre à l'étape B.

3. Procédé selon la revendication 2 dans lequel la teneur en EB de la première fraction est supérieur d'au moins 1,0% par rapport à la seconde fraction.

4. Procédé selon l'une quelconque des revendications 1 à 3 dans lequel la colonne de distillation mise en oeuvre à l'étape B comprend entre 30 et 80 plateaux théoriques.

5. Procédé selon l'une quelconque des revendications précédentes dans lequel l'étape B de distillation met en oeuvre une colonne comprenant une paroi interne.

6. Procédé selon l'une quelconque des revendications précédentes comprenant une étape C d'isomérisation en phase vapeur de la première fraction (B2) issue d'une étape de fractionnement contenant de l'EB, de l'OX et du MX.

7. Procédé selon l'une quelconque des revendications précédentes comprenant une étape D d'isomérisation en phase liquide de la fraction (B31) contenant de l'OX et du MX issue de la seconde colonne de distillation (B_C4) mise en oeuvre à l'étape de fractionnement.

8. Procédé selon la revendication 6 dans lequel l'étape C d'isomérisation en phase vapeur est mise en oeuvre à une température supérieure à 300°C, une pression inférieure à 4,0 MPa, une vitesse spatiale inférieure à 10,0 h⁻¹, rapport molaire hydrogène sur hydrocarbure inférieur à 10,0, et en présence d'un catalyseur comportant au moins une zéolithe présentant des canaux dont l'ouverture est définie par un anneau à 10 ou 12 atomes d'oxygène (10 MR ou 12 MR), et au moins un métal du groupe VIII de teneur comprise entre 0,1 et 0,3% poids.

9. Procédé selon la revendication 8 dans lequel le catalyseur mise en œuvre à l'étape C comprend de 1 à 70% poids d'une zéolithe de type structural EUO, ladite zéolithe comprenant du silicium et au moins un élément T choisi de préférence parmi l'aluminium et le bore dont le rapport Si/T est compris entre 5 et 100.

10. Procédé selon l'une quelconque des revendications 7 dans lequel l'étape D d'isomérisation en phase liquide est mise en œuvre à une température inférieure à 300°C, une pression inférieure à 4,0 MPa, une vitesse spatiale inférieure à 5,0 h⁻¹ et de préférence comprise entre 2,0 et 4,0 h⁻¹, et en présence d'un catalyseur comportant au moins une zéolithe présentant des canaux dont l'ouverture est définie par un anneau à 10 ou 12 atomes d'oxygène (10 MR ou 12 MR), de préférence ladite zéolithe est de type ZSM-5.

## Patentansprüche

1. Verfahren zur Herstellung von para-Xylol ausgehend von einem Einsatzstoff, der Xylole, Ethylbenzol und C9+-Kohlenwasserstoffe enthält, umfassend
- einen einzigen Schritt A der Trennung des eingesetzten Einsatzstoffs in einem simulierten Fließbett mit einem Zeolith als Adsorptionsmittel und einem Desorptionsmittel bei einer Temperatur zwischen 20 und 250 °C unter einem Druck zwischen 1,0 und 2,2 MPa und mit einem Volumenverhältnis von Desorptionsmittel zu Einsatzstoff in der Einheit zur Trennung in einem simulierten Fließbett zwischen 0,4 und 2,5;
wobei der Schritt A es ermöglicht, Folgendes zu erhalten:
▪ eine erste Fraktion (A1), die ein Gemisch von Para-Xylol und Desorptionsmittel enthält, und
▪ mindestens eine zweite Fraktion (A2 oder A21), die Ethylbenzol (EB), ortho-Xylol (OX) und meta-Xylol (MX) und Desorptionsmittel enthält,
- einen Schritt B der Fraktionierung der aus dem Schritt A stammenden zweiten Fraktion durch Destillation in mindestens einer ersten Destillationskolonne (B_C3), was es ermöglicht, 3 Fraktionen zu erhalten:
▪ eine erste Fraktion (B2), die EB, OX und MX enthält,
▪ eine zweite Fraktion (B3), die OX und MX enthält, und
▪ eine dritte Fraktion (B42), die Desorptionsmittel enthält,
wobei
der Schritt A der Trennung es ermöglicht, eine an EB abgereicherte dritte Fraktion A22, die ein Gemisch von MX, OX und Desorptionsmittel enthält, zu erhalten, wobei die Fraktionen A21 und A22 dem Schritt B der Fraktionierung zugeführt werden,
die aus Schritt A stammende dritte Fraktion (A22) in eine zweite Destillationskolonne (B-C4) eingetragen wird, was es ermöglicht, eine desorptionsmittelfreie Fraktion (B31), die MX und OX enthält, und eine Fraktion B43, die aus Desorptionsmittel besteht, zu erhalten,
die aus Schritt B der Fraktionierung stammende zweite Fraktion (B3) in eine zweite Destillationskolonne (B-C4) eingetragen wird, was es ermöglicht, eine desorptionsmittelfreie Fraktion (B31), die MX und OX enthält, und eine Fraktion B43, die aus Desorptionsmittel besteht, zu erhalten,
die aus Schritt A stammende dritte Fraktion (A22) unterhalb des seitlichen Einleitungspunkts der aus der ersten Destillationskolonne (B-C3) stammenden zweiten Fraktion (B3) in die zweite Destillationskolonne (B-C4) eingetragen wird.

2. Verfahren nach Anspruch 1, wobei die aus Schritt B stammende erste Fraktion (B2) einen höheren EB-Gehalt als die aus der in Schritt B verwendeten ersten Kolonne (B_C3) stammende zweite Fraktion (B3) aufweist.

3. Verfahren nach Anspruch 2, wobei der EB-Gehalt der ersten Fraktion mindestens 1,0 % höher ist als der der zweiten Fraktion.

4. Verfahren nach einem der Ansprüche 1 bis 3, wobei die in Schritt B verwendete Destillationskolonne 30 bis 80 theoretische Trennstufen umfasst.

5. Verfahren nach einem der vorhergehenden Ansprüche, wobei im Schritt B der Destillation eine Kolonne mit einer Innenwand verwendet wird.

6. Verfahren nach einem der vorhergehenden Ansprüche, umfassend einen Schritt C der Isomerisierung der aus einem Schritt der Fraktionierung stammenden ersten Fraktion (B2), die EB, OX und MX enthält, in der Dampfphase.

7. Verfahren nach einem der vorhergehenden Ansprüche, umfassend einen Schritt D der Isomerisierung der aus der im Schritt der Fraktionierung verwendeten zweiten Destillationskolonne (B_C4) stammenden Fraktion (B31), die OX und MX enthält, in der Flüssigphase.

8. Verfahren nach Anspruch 6, wobei der Schritt C der Isomerisierung in der Dampfphase bei einer Temperatur von mehr als 300 °C, einem Druck von weniger als 4,0 MPa, einer Raumgeschwindigkeit von weniger als 10,0 h⁻¹, einem Molverhältnis von Wasserstoff zu Kohlenwasserstoff von weniger als 10,0 und in Gegenwart eines Katalysators, der mindestens einen Zeolith mit Kanälen, deren Öffnung durch einen Ring mit 10 oder 12 Sauerstoffatomen definiert ist (10 MR oder 12 MR), und mindestens ein Metall der Gruppe VIII in einem Gehalt zwischen 0,1 und 0,3 Gew.-% umfasst, durchgeführt wird.

9. Verfahren nach Anspruch 8, wobei der in Schritt C verwendete Katalysator 1 bis 70 Gew.-% eines Zeoliths vom EUO-Strukturtyp umfasst, wobei der Zeolith Silicium und mindestens ein Element T, das vorzugsweise aus Aluminium und Bor ausgewählt ist, umfasst, wobei das Si/T-Verhältnis zwischen 5 und 100 liegt.

10. Verfahren nach einem der Ansprüche 7, wobei der Schritt D der Isomerisierung in der Flüssigphase bei einer Temperatur von weniger als 300 °C, einem Druck von weniger als 4,0 MPa, einer Raumgeschwindigkeit von weniger als 5,0 h⁻¹ und vorzugsweise zwischen 2,0 und 4,0 h⁻¹, und in Gegenwart eines Katalysators, der mindestens einen Zeolith mit Kanälen, deren Öffnung durch einen Ring mit 10 oder 12 Sauerstoffatomen definiert ist (10 MR oder 12 MR), umfasst, durchgeführt wird, wobei der Zeolith vorzugsweise vom ZSM-5-Typ ist.

## Claims

1. Process for the production of para-xylene from a feedstock containing xylenes, ethylbenzene and C9+ hydrocarbons, comprising:
- a single stage A of separation in a simulated moving bed of said feedstock employed with a zeolite as adsorbent and a desorbent, at a temperature of between 20 et 250°C, under a pressure of between 1.0 and 2.2 MPa, and with a ratio by volume of the desorbent to the feedstock in the simulated moving bed separation unit of between 0.4 and 2.5;
said stage A making it possible to obtain:
▪ a first fraction (A1) containing a mixture of para-xylene and of desorbent, and
▪ at least one second fraction (A2 or A21) containing ethylbenzene (EB), ortho-xylene (OX) and meta-xylene (MX) and desorbent,
- a stage B of fractionation by distillation in at least one first distillation column (B-C3) of said second fraction resulting from stage A, making it possible to obtain 3 fractions:
▪ a first fraction (B2) containing EB, OX and MX,
▪ a second fraction (B3) containing OX and MX, and
▪ a third fraction (B42) containing desorbent,
in which:
the separation stage A makes it possible to obtain a third fraction A22 depleted in EB containing a mixture of MX, OX and desorbent, said fractions A21 and A22 being sent into said fractionation stage B,
the third fraction (A22) resulting from stage A is introduced into a second distillation column (B-C4), making it possible to obtain a fraction (B31) devoid of desorbent containing MX and OX, and a fraction B43 consisting of desorbent,
the second fraction (B3) is introduced at the fractionation stage B into a second distillation column (B-C4), making it possible to obtain a fraction (B31) devoid of desorbent containing MX and OX, and a fraction B43 consisting of desorbent,
the third fraction (A22) resulting from stage A is introduced into the second distillation column (B-C4) below the point of sidestream injection of the second fraction (B3) resulting from the first distillation column (B-C3).

2. Process according to Claim 1, in which the first fraction (B2) resulting from stage B exhibits a greater EB content than the EB content of the second fraction (B3) resulting from the first column (B-C3) employed in stage B.

3. Process according to Claim 2, in which the EB content of the first fraction is greater by at least 1.0% with respect to the second fraction.

4. Process according to any one of Claims 1 to 3, in which the distillation column employed in stage B comprises between 30 and 80 theoretical plates.

5. Process according to any one of the preceding claims, in which the distillation stage B employs a column comprising an internal wall.

6. Process according to any one of the preceding claims, comprising a stage C of vapour-phase isomerization of the first fraction (B2) resulting from a fractionation stage containing EB, OX and MX.

7. Process according to any one of the preceding claims, comprising a stage D of liquid-phase isomerization of the fraction (B31) containing OX and MX resulting from the second distillation column (B-C4) employed in the fractionation stage.

8. Process according to Claim 6, in which the vapour-phase isomerization stage C is carried out at a temperature of greater than 300°C, a pressure of less than 4.0 MPa, a space velocity of less than 10.0 h⁻¹, a hydrogen to hydrocarbon molar ratio of less than 10.0, and in the presence of a catalyst comprising at least one zeolite exhibiting channels, the opening of which is defined by a ring having 10 or 12 oxygen atoms (10 MR or 12 MR), and at least one metal from group VIII at a content of between 0.1% and 0.3% by weight.

9. Process according to Claim 8, in which the catalyst employed in stage C comprises from 1% to 70% by weight of a zeolite of EUO structural type, said zeolite comprising silicon and at least one element T preferably chosen from aluminium and boron, the Si/T ratio of which is of between 5 and 100.

10. Process according to any one of Claims 7, in which the liquid-phase isomerization stage D is carried out at a temperature of less than 300°C, a pressure of less than 4.0 MPa, a space velocity of less than 5.0 h⁻¹ and preferably of between 2.0 and 4.0 h⁻¹, and in the presence of a catalyst comprising at least one zeolite exhibiting channels, the opening of which is defined by a ring having 10 or 12 oxygen atoms (10 MR or 12 MR); preferably, said zeolite is of ZSM-5 type.
